(19) Europäisches Patentamt
European Patent Office
Office européen des brevets

(11) Publication number: **0 080 012 B1**

(12) **EUROPEAN PATENT SPECIFICATION**

(45) Date of publication of patent specification: **09.04.86**

(21) Application number: **82105188.5**

(22) Date of filing: **04.07.79**

(60) Publication number of the earlier application in accordance with Art. 76 EPC: **0 007 070**

(51) Int. Cl.[4]: **C 07 D 223/16** // A61K31/55, C07C43/225, C07C43/23, C07C57/30, C07C121/68, C07D405/12, C07D409/12

# (54) 6-Chloro-3-methyl-2,3,4,5-tetrahydro-1H-3-benzazepine, its acid addition salts and its use as an intermediate.

(30) Priority: **07.07.78 US 922613**

(43) Date of publication of application: **01.06.83 Bulletin 83/22**

(45) Publication of the grant of the patent: **09.04.86 Bulletin 86/15**

(84) Designated Contracting States: **AT BE CH DE FR GB IT LU NL SE**

(56) References cited:
**GB-A-1 268 243**
**US-A-2 520 264**
**US-A-3 716 639**
**US-A-3 752 892**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Holden, Kenneth George**
**425 Beechwood Avenue**
**Haddonfield New Jersey 08033 (US)**
Inventor: **Kaiser, Carl**
**1105 Sylvan Drive**
**Haddon Heights New Jersey 08035 (US)**

(74) Representative: **Vossius Vossius Tauchner Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 080 012 B1

**Description**

This application is a divisional application of EP—A—79 10 2279.1 (European Patent 0 007 070).

The invention relates to the novel 6-chloro-3-methyl - 2,3,4,5 - tetrahydro - 1H - 3 - benzazepine which is a valuable starting material for the preparation of certain mercapto substituted-2,3,4,5-tetrahydro-1H-3-benzazepines having pharmacodynamic activity. More specifically, these compounds have dopamine receptor blocking activity and therefore are useful as antipsychotic and antiemetic agents. The antipsychotic activity is similar to that of chlorpromazine.

The compound of the invention, namely 6-chloro - 3 - methyl - 2,3,4,5 - tetrahydro-1H-3-benzazepine, is prepared by methods known in the art. For example, US—A—3,752,892 discloses the preparation of 6-chloro-2,3,4,5-tetrahydro-1H-3-benzazepine by cyclization of an N-(2-haloethyl)-phenethylamine employing a Lewis acid such as aluminum chloride to obtain 2,3,4,5-tetrahydro-1H-3-benzazepine which is subsequently chlorinated. Also, US—A—3,716,639 discloses the pre-preparation of 7- chloro - 2,3,4,5 - tetrahydro - 1H-3 - benzazepine by cyclisation of N - (2 - chloroethyl) - p - chlorophenethylamine hydrochloride employing aluminium chloride.

The mercapto substituted-2,3,4,5-tetrahydro-1H-3-benzazepines are represented by the following general structural formula (I):

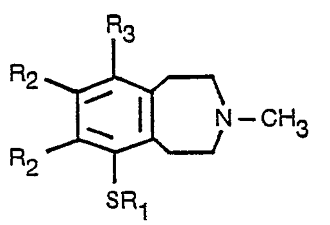

I

wherein

$R_1$ represents cyclohexyl, thienyl, thienylmethyl, furyl or furylmethyl, phenyl, or m- or p-substituted phenyl with the substituent being trifluoromethyl, chloro, methoxy, methyl, fluoro, nitro or hydroxy;

$R_2$ represents hydrogen or hydroxy, with the proviso that if one $R_2$ is hydroxy, the other $R_2$ is hydrogen and

$R_3$ represents hydrogen, chloro or bromo.

US—A—3,671,519 and 3,483,185 name "2,3,4,5-tetrahydro-8-methylmercapto-1H-3-benzazepine" as a starting material, however neither of these or equivalent prior art discloses the mercapto substituents of formula (I) above in a 3-benzazepine series.

The compounds of formula (I) wherein $R_2$ and $R_3$ are all hydrogen are conveniently prepared from the chloro substituted benzazepine by reaction with for example n-butyl lithium followed by the appropriately substituted disulfide. Introduction of an $R_3$ substituent other than hydrogen is accomplished for example by nitration of a chloro substituted benzazepine, displacement of the chlorine by the appropriately substituted mercaptan, followed by reduction of the nitro group, subsequent diazotization of the amine and conversion of the diazonium salt to the appropriate $R_3$ substituted derivative. Similarly compounds of formula I wherein one $R_2$ is hydroxy and $R_3$ is hydrogen are obtained from the above described amino substituted benzazepine by diazotization followed by treatment with aqueous sulfuric acid. It will be obvious to one skilled in the art that other combinations of these basic reactions will give compounds of formula I wherein one $R_2$ is hydroxy and $R_3$ is other than hydrogen, as illustrated in the examples below.

The dopamine receptor blocking activity of the compounds of formula (I) is demonstrated by antagonism of avoidance acquisition in rats and/or block of the effects of dopamine on dopamine sensitive adenylate cyclase in rat striatal homogenate. Central dopamine receptor blocking activity is a measure of potential antipsychotic activity. In the pharmacological procedure used to measure antagonism of avoidance acquisition, naive male rats are given either a test compound or saline at a suitable time period prior to testing. The rats are then placed in a dark soundproof box with a grid floor through which footshock is delivered. Trials begin at 30-second intervals. The beginning of each trial is signaled by a light and a buzzer which continues for 10 seconds, at which time footshock is added for an additional 15 seconds. In each trial a single lever press by the animal terminates the sequence. Evaluation of drug activity is based on the number of trials in which the animals fail to avoid or fail to escape footshock during the last 40 trials of a 100 trial, 50-minute session. The $ED_{50}$ is defined as that dose of drug calculated to reduce the number of avoidance responses during the last 40 trials to 50% of the (pooled) control value.

As an example of the antipsychotic activity of a compound of formula (I), the $Ed_{50}$ value in mg/kg, i.p. obtained from testing the indicated compound in the above procedure is as follows:

3-methyl-6-phenylthio-2,3,4,5-tetrahydro-1H-3-benzazepine, $ED_{50}$ 1.6;

For comparison, chlorpromazine has an Avoidance $ED_{50}$ of 1.5 mg/kg, i.p.

The following examples illustrate the preparation of specific compounds. Those skilled in the art will appreciate that other modifications of the synthetic procedures described may also be employed to prepare the compounds of formula (I).

Example 1

To a stirred mixture of 400 g of concentrated sulfuric acid and 100 g of concentrated nitric acid at 0—5°C is added, in portions, 19.6 g (0.1 mole) of 6 - chloro-3-methyl-2,3,4,5-tetrahydro-1H-3-benzazepine. The solution is stirred at 0—5°C for 2.5 hours, then it is poured *cautiously* into 1.5 liters of ice/water. The solution is made basic by addition of excess sodium hydroxide, then it is extracted with ether. After being washed several times with water the extract is dried and concentrated. The

resulting mixture of approximately equal parts of 6-chloro-3-methyl-9-nitro-2,3,4,5-tetrahydro-1H-3-benzazepine and 6-chloro-3-methyl-7-nitro-2,3,4,5-tetrahydro-1H-3-benzazepine is separated by chromatographic methods.

To a stirred solution of 11.0 g (0.1 mole) of thiophenol in 200 ml of dimethylformamide at 0—10°C., under an atmosphere of nitrogen, is added *cautiously*, in portions, 4.65 g (0.11 mole) of a 57% dispersion of sodium hydride in mineral oil. The resulting solution is stirred for 15 minutes at 25°C and then a solution of 24.1 g (0.1 mole) of 6-chloro-3-methyl-9-nitro-2,3,4,5-tetrahydro-1H-3-benzazepine in 50 ml of dimethylformamide is added dropwise. The reaction mixture is heated at 100°C for 2 hours, then it is cooled to 25°C and poured into ice/water. The resulting solid is filtered, air-dried and recrystallized from ethyl acetate-hexane to give 3-methyl-9-nitro-6-phenylthio-2,3,4,5-tetrahydro-1H-3-benzazepine.

To a solution of 15.7 g (0.05 mole) of 3-methyl-9-nitro-6-phenylthio-2,3,4,5-tetrahydro-1H-3-benzazepine in 350 ml of ethanol and 125 ml of water is added, in portions 35 g (0.2 mole) of sodium hydrosulfite. The mixture is stirred and refluxed for 16 hours, then an additional 52 g (0.3 mole) of sodium hydrosulfite is added and refluxing is continued for 30 hours, allowing about one-half of the solvent to distill from the reaction during the last hour. The mixture is cooled, diluted with water, made alkaline with ammonium hydroxide and extracted with ethyl acetate. After being dried, the extract is concentrated to give 9-amino-3-methyl-6-phenylthio-2,3,4,5-tetrahydro-1H-3-benzazepine which is purified by chromatography. A solution of the base is ethanol is treated with an excess of hydrogen chloride. Following addition of ether crystalline 9-amino-3-methyl-6-phenylthio-2,3,4,5-tetrahydro-1H-3-benzazepine dihydrochloride is obtained.

Alternatively the 9-nitro compound is hydrogenated in ethanol solution with 5% palladium-on-carbon at 50 p.s.i. for 2 hours to give the 9-amino derivative.

To a stirred suspension of 17.9 g (0.05 mole) of 9-amino-3-methyl-6-phenylthio-2,3,4,5-tetrahydro-1H-3-benzazepine dihydrochloride in 50 ml of water and 50 ml of concentrated hydrochloric acid at 0—5°C is added dropwise a solution of 4.2 g (0.06 mole) of sodium nitrite in 25 ml of water. After being stirred at 0—5°C for 30 minutes, the resulting diazonium solution is added to a solution of 6.0 g (0.06 mole) of cuprous chloride in 25 ml of concentrated hydrochloric acid. The mixture is stirred for 16 hours at 25°C, then it is warmed to 60—80°C for 1 hour. After being cooled to 15—20°C, the mixture is made alkaline and extracted with ether. The ether extract is dried over magnesium sulfate and concentrated to leave 9-chloro-3-methyl-6-phenylthio-2,3,4,5-tetrahydro-1H-3-benzazepine which is purified by chromatography or by recrystallization of appropriate acid addition salts; hydrochloride salt m.p. 231—232°C.

## Example 2

To a stirred solution of 114 ml of water and 15 ml of concentrated sulfuric acid at 60—70°C is added 23.2 g (0.082 mole) of 9-amino-3-methyl-6-phenylthio-2,3,4,5-tetrahydro-1H-3-benzazepine. The resulting suspension is stirred vigorously and cooled to 0—5°C. To this suspension is added 6.3 g (0.091 mole) of sodium nitrite in 10 ml of water at a rate such that the temperature does not exceed 5°C. The resulting diazonium solution is added dropwise to a boiling solution of 200 g of cuprous sulfate and 300 ml of water. After being refluxed for 15 minutes the solution is cooled, a trace of ascorbic acid is added and the pH is adjusted to 7.0 with ammonium hydroxide. The mixture is extracted with ethyl acetate. After being dried the extract is concentrated to afford 9-hydroxy-3-methyl-6-phenylthio-2,3,4,5-tetrahydro-1H-3-benzazepine. Purification is accomplished by chromatography or by recrystallization of an appropriate acid addition salt.

## Example 3

Following the procedures outlined in Example 7, the isomeric 6-chloro-3-methyl-7-nitro-2,3,4,5-tetrahydro-1H-3-benzazepine is treated with sodium thiophenolate to give the 6-phenylthio intermediate and the nitro group is reduced with sodium hydrosulfite. To a solution of 2.6 g (0.0125 mole) of the resulting 7-amino-3-methyl-6-phenylthio-2,3,4,5-tetrahydro-1H-3-benzazepine in 25 ml of 3 N sulfuric acid at 0—3°C, a solution of sodium nitrite (1 g in 5 ml of water) is added dropwise until a positive test for nitrous acid is obtained. Excess nitrous acid is decomposed by adding 0.2 to 0.3 g of urea and stirring for 10 minutes. The diazonium solution is added dropwise with stirring to 200 ml of 50% sulfuric acid at 70°C and maintained at 70°C until all of the diazonium salt is decomposed. On cooling the warm solution in an ice bath a crystalline precipitate is formed. After being chilled for 30 minutes at 0°C, the mixture is filtered. The solid is washed with a small volume of ice/water. Recrystallization affords 7-hydroxy-3-methyl-6-phenylthio-2,3,4,5-tetrahydro-1H-3-benzazepine sulfate.

## Example 4

A suspension of 12.0 g (0.05 mole) of 6-chloro-3-methyl-9-nitro-2,3,4,5-tetrahydro-1H-3-benzazepine, 100 ml of ethanol and 0.2 g of platinum dioxide is hydrogenated on a Parr apparatus at 25°C and an initial hydrogen pressure of 60 p.s.i. After the rapid hydrogen uptake is completed, the mixture is filtered and the filtrate is concentrated in vacuo to give 9-amino-6-chloro-3-methyl-2,3,4,5-tetrahydro-1H-3-benzazepine.

A mixture of 10.5 g (0.05 mole) of 9-amino-6-chloro-3-methyl-2,3,4,5-tetrahydro-1H-3-benzazepine and 50 ml of acetic anhydride is stirred and heated at 60—65°C for 4 hours. The resulting solution is poured into ice/water and stirred at 25°C for 16 hours, then it is made alkaline by addition of sodium hydroxide at 5—10°C. The

precipitate is immediately filtered to give 9-acetamido - 6 - chloro - 3 - methyl - 2,3,4,5 - tetrahydro - 1H - 3 - benzazepine.

To a stirred mixture of 200 g of concentrated sulfuric acid and 50 g of concentrated nitric acid at 0—5°C is added, in portions, 12.6 g. (0.05 mole) of 9-acetamido-6-chloro-3-methyl-2,3,4,5-tetrahydro-1H-3-benzazepine. The solution is stirred at 0—5°C for 2 hours and then it is poured *cautiously* into 500 ml of ice/water. The solution is made alkaline with sodium hydroxide. After being stirred at 25°C for 16 hours, the mixture is filtered to give 9-amino-6-chloro-3-methyl-8-nitro-2,3,4,5-tetrahydro-1H-3-benzazepine.

A solution of 100 ml of sulfuric acid and 50 ml of water is cooled to −10°C and maintained at this temperature while 3.7 g (0.054 mole) of sodium nitrite is added in small portions over a period of about 15 minutes. Cold 50% hypophosphorous acid 19.3 ml, (0.186 mole) is added over a period of 10—15 minutes, the temperature still being maintained at −10°C. A solution of 5.1 g (0.02 mole) of 9-amino-6-chloro-3-methyl-8-nitro-2,3,4,5-tetrahydro-1H-3-benzazepine in 100 ml of glacial acetic acid is then added to the stirred diazonium solution dropwise during a period of 1 hour as the temperature is maintained at −10°C. Stirring is continued for 2 hours allowing the temperature to rise to 5°C. The solution is maintained at this temperature in a hood for 36 hours, then the solution is steam distilled to remove acetic acid. The residual liquid is cooled and sodium hydroxide is cautiously added with stirring. The crystalline 6-chloro-3-methyl-8-nitro-2,3,4,5-tetrahydro-1H-3-benzazepine is filtered. It may be purified by chromatography or recrystallization from ethyl acetatehexane.

A stirred solution of 62 ml of 0.9 M *n*-butyl lithium (0.056 mole) in tetrahydrofuran, under nitrogen, is cooled to −70°C and a solution of 2.4 g (0.01 mole) of 6-chloro-3-methyl-8-nitro-2,3,4,5-tetrahydro-1H-3-benzazepine in 25 ml of tetrahydrofuran is added during a period of 30 minutes. The solution is stirred at −70°C for 30 minutes and then a solution of 13.5 g (0.06 mole) of diphenyldisulfide in 40 ml of tetrahydrofuran is added dropwise. After being stirred at −70°C for 1 hour the solution is poured into 500 ml of ice/water containing excess hydrochloric acid. The mixture is extracted with ethyl acetate and then the aqueous phase is made alkaline with 10 N sodium hydroxide to precipitate 3-methyl-8-nitro-6 - phenylthio - 2,3,4,5 - tetrahydro - 1H - 3 - benzazepine. The product is filtered and recrystallized from ethyl acetate-hexane or aqueous ethanol.

Following the procedures outlined in Examples 1 and 2, the 3-methyl-8-nitro-6-phenylthio-2,3,4,5-tetrahydro-1H-3-benzazepine is reduced with sodium hydrosulfite and the corresponding 8-amino derivative is diazotized and then heated with cuprous sulfate/sulfuric acid to yield 8-hydroxy - 3 - methyl - 6 - phenylthio - 2,3,4,5 - tetra-hydro - 1H - 3 - benzazepine.

Example 5

Following the procedures outlined in Example 4, 9-amino-6-chloro-3-methyl-8-nitro-2,3,4,5-tetrahydro-1H-3-benzazepine is treated with *n*-butyl lithium followed by diphenyldisulfide to give the corresponding 6-phenylthio derivative which is diazotized and then reacted with cuprous chloride and hydrochloric acid to give 9-chloro-8-nitro--3-methyl - 6-phenylthio-2,3,4,5-tetrahydro-1H-3-benzazepine. The latter is reduced with sodium hydrosulfite and the resulting 8-amino derivative is diazotized and then treated with cuprous sulfate/sulfuric acid to furnish 9-chloro-8-hydroxy-3-methyl - 6 - phenylthio - 2,3,4,5 - tetrahydro - 1H-3 - benzazepine.

Example 6

To a solution of 1.3 g (0.02 mole) of potassium hydroxide in 20 ml of water was added 2.9 g (0.022 mole) of p-fluorothiophenol in 20 ml of ethanol. The mixture was refluxed for 1 hour and 4.8 g (0.02 mole) of 6-chloro-3-methyl-9-nitro-2,3,4,5-tetrahydro-1H-3-benzazepine (prepared as in Example 1) in 20 ml of ethanol was added. The resulting solution was refluxed for 4.5 hours and allowed to cool. A red oil was decanted from the reaction mixture, dissolved in ethyl acetate and washed with saturated sodium chloride solution and 10% sodium hydroxide solution. The dried ethyl acetate solution was evaporated to give 5.3 g of 6-(p-fluorophenylthio)-3-methyl-9-nitro-2,3,4,5-tetrahydro-1H-3-benzazepine.

A mixture of 4.3 g (0.0135 mole) of the above prepared 9-nitro derivative dissolved in 100 ml of ethanol, 50 ml of 1N sulfuric acid and 0.4 g of 5% palladium-on-carbon in 50 ml of ethanol was hydrogenated at 60 p.s.i. for 2 hours. The catalyst was filtered from the reaction mixture and the filtrate was evaporated. The residue was dissolved in a minimum amount of ethanol to which ethereal hydrogen chloride was added. The solid was filtered to give 1.5 g of 9-amino-6-(p-fluorophenylthio)-3-methyl-2,3,4,5-tetrahydro-1H-3-benzazepine dihydrochloride.

Following the procedure outlined in Example 1, the 9-amino-3-benzazepine dihydrochloride (1.25 g) was diazotized with sodium nitrite in water and concentrated hydrochloric acid and then treated with cuprous chloride to yield, after purification on silica, 9-chloro-6-(p-fluoro-phenylthio) - 3 - methyl - 2,3,4,5 - tetrahydro - 1H - 3 - benzazepine; hydrochloride salt m.p. 212—213°C.

**Claims**

1. 6-Chloro-3-methyl-2,3,4,5-tetrahydro-1H-3-benzazepine.

2. Use of the compound of claim 1 of preparing mercapto substituted -2,3,4,5-tetrahydro-1H-3-benzazepines of formula I:

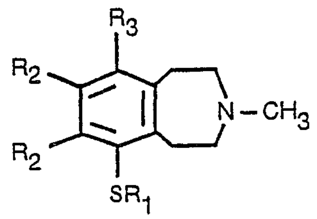

I

wherein

$R_1$ represents cyclohexyl, thienyl, thienylmethyl, furyl or furylmethyl, phenyl, or m- or p-substituted phenyl with the substituent being trifluoromethyl, chloro, methoxy, methyl, fluoro, nitro or hydroxy;

$R_2$ represents hydrogen or hydroxy, with the proviso that if one $R_2$ is hydroxy, the other $R_2$ is hydrogen; and

$R_3$ represents hydrogen, chloro or bromo.

**Patentansprüche**

1. 6-Chlor-3-methyl-2,3,4,5-tetrahydro-1H-3-benzazepin.

2. Verwendung der Verbindung nach Anspruch 1 zur Herstellung von Mercapto-substituierten 2,3,4,5-Tetrahydro-1H-3-benzazepinen der allgemeinen Formel I:

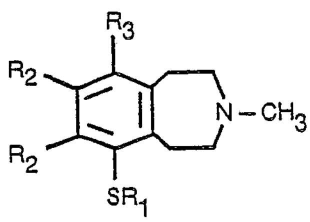

I

in der

$R_1$ eine Cyclohexyl-, Thienyl-, Thienylmethyl-, Furyl- oder Furylmethyl-, Phenyl- oder eine m- oder p-substituierte Phenylgruppe bedeutet, wobei der Substituent eine Trifluormethyl-, Chlor-, Methoxy-, Methyl-, Fluor-, Nitro- oder Hydroxylgruppe ist;

$R_2$ ein Wasserstoffatom oder eine Hydroxylgruppe bedeutet, mit der Maßgabe, daß, wenn der eine $R_2$-Rest eine Hydroxylgruppe ist, der andere Rest $R_2$ ein Wasserstoffatom ist; und

$R_3$ ein Wasserstoff-, Chlor- oder Bromatom bedeutet.

**Revendications**

1. 6-Chloro-3-méthyl-2,3,4,5-tétrahydro-1H-3-benzazépine.

2. Utilisation du composé de la revendication 1 pour le préparation des mercapto-2,3,4,5-tétrahydro-1H-3-benzazépine de formule I:

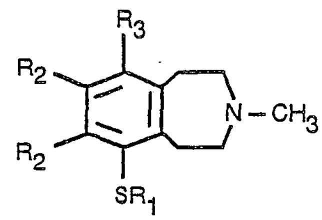

I

dans laquelle

$R_1$ représente un groupe cyclohexyle, thiényle, thiénylméthyle, furyle ou furylméthyle, phényle ou phényle m- ou p- substitué par le substituant étant un groupe trifluorométhyle, chloro, méthoxy, méthyle, fluoro, nitro ou hydroxy.

$R_2$ représente de l'hydrogène ou un groupe hydroxy à condition que si un $R_2$ représente un groupe hydroxy, l'autre $R_2$ représente de l'hydrogène et

$R_3$ représente de l'hydrogène, un groupe chloro ou bromo.